# EUROPÄISCHE PATENTSCHRIFT

(11) **EP 2 440 083 B1**
(45) Veröffentlichungstag und Bekanntmachung des Hinweises auf die Patenterteilung: **30.04.2014**
(21) Anmeldenummer: 09737326.0
(22) Anmeldetag: 19.10.2009
(51) Int. Cl.: A43B 7/14, A43B 17/00, A61F 5/14

(54) **HALBFABRIKAT ZUR FERTIGUNG EINER INNENSOHLE ODER EINLEGESOHLE, SOWIE DARAUS GEFERTIGTE INNENSOHLE ODER EINLEGESOHLE**
HALF-FINISHED PRODUCT TO PRODUCE INNSOLES OR SOCKS, AS WELL AS THE INNSOLE OR SOCK PRODUCED WITH IT
PRODUIT SEMI-FINI POUR LA RÉALISATION D'UNE SEMELLE INTÉRIEURE OU UNE PREMIERE DE MONTAGE, AINSI QUE LA SEMELLE OU LA PREMIERE RÉALISÉE AVEC CELUI-CI

(30) Priorität: 09.06.2009 CH 885092009; 12.06.2009 CH 918092009; 25.08.2009 CH 13122009
(43) Veröffentlichungstag der Anmeldung: 18.04.2012
(73) Patentinhaber: Mafag-Reflexa AG, 8302 Kloten (CH)
(72) Erfinder: EVERZ VAZ, Samantha, CH-8008 Zürich (CH)
(74) Vertreter: Schneider Feldmann AG Patent- und Markenanwälte
(86) Internationale Anmeldenummer: PCT/CH2009/000335
(87) Internationale Veröffentlichungsnummer: WO 2010/142047

(56) Entgegenhaltungen:
- EP-B1- 1 245 167
- WO-A1-88/06009
- DE-A1- 2 024 534
- DE-A1- 3 508 582
- DE-U- 1 904 447
- US-A- 4 823 799

## Beschreibung

Die vorliegende Erfindung betrifft ein Halbfabrikat zur Fertigung einer Innensohle, bestehend ais einer dem Umriss eines Schuhs entsprechenden, elastischen Schaumstoffmateriallage die auf ihrer oberen Seite, von der Grundflaeche ausgehend, mehrere ebenfalls aus demselben elastischen Schaumstoffmaterial bestehende, integral angeformte Erhebungen im Bereich von Reflexzonen der Fusssohle aufweist. Die Erfindung betrifft auch eine Innensohle oder Einlegesohle die unter Verwendung des erfindungsgemässen Halbfabrikates gefertigt ist.

Unter dem Begriff Halbfabrikat einer Innensohle wird hier jedes einem Umriss eines Schuhs entsprechenden Grundmaterial verstanden, das, wenn mindestens zusammen mit einer Deckschicht versehen, eine Innensohle formt, die die nötige Härte aufweist.

Unter dem Begriff Innensohle wird hier einerseits eine mit einer Schuhsohle fest verbundene Innensohle verstanden, wie auch eine lose darauf aufgelegte Sohle, die auch als Einlegesohle bezeichnet wird.

Innensohlen, welche mit zur Reflexzonenmassage geeigneten Erhebungen aufweisen, sind in verschiedensten Ausführungsformen bekannt. Die Mehrzahl solcher Innensohlen weisen eine über die gesamte Oberfläche angeordnet genoppte Auflagefläche auf, wobei die Oberflächenstruktur der Innensohle durch die Gesamtheit der Noppen gebildet wird. Innensohlen dieser Art zeigen beispielsweise: die EP-0'225'285, sowie die DE 2 024 534 und die US-4,760,655 A. Während im letzteren Fall gleich wie bei der CH-686'062 A die Noppenhöhe über die gesamte Fläche der Einlegesohle gleichbleibend gestaltet ist, sind andere Innensohlen, wie beispielsweise gemäss der WO 85/04786 mit Noppen gestaltet, deren Höhe variiert und die entsprechend praktisch ein Fussbett bilden. Innensohlen dieser Art bewirken möglicherweise eine Überreizung der Fusssohle, womit die gewünschte Fussreflexzonenmassage nicht mehr die erwünschte Wirkung zeigt. Vielmehr kann eine Ueberreizung zu Blockaden der Nervenleitungen und zu schädlichen Folgen führen. Korrekterweise verweisen entsprechende Anbieter darauf, dass solches Schuhwerk nur während 1-2 Stunden getragen werden sollte.

Aus der WO 88/06009 ist eine Sohle bekannt, die mit einem Rillemuster versehen ist, uber der eine Folie mit geschlossener Porenstruktur angebracht ist.

Bezüglich der Herstellung von Innensohlen mit Erhebungen aus elastischem Schaumstoffmarerial sind zwei Konstruktionsprinzipien bekannt. Im einen Fall wird von einer härteren Grundschicht ausgegangen und darauf kissenförmige Erhebungen aufgeklebt. Dies ist eine äusserst aufwendige und teure Fertigungsweise. Diese Fertigungsweise ist beispielsweise aus der bereits erwähnten US-4,760,655 A bekannt. Damit die Erhebungen noch eine relevante Massagewirkung erzielen, müssen sie relativ stark gegenüber der Grundfläche vorstehen. Wegen der genoppten Deckschicht, die darüber angebracht wird und sich über die gesamte Innensohle erstreckt, wird praktisch unerwünschterweise die Massage in den härteren, nicht erhöhten Bereichen stärker wahrgenommen als in den erhöhten Bereichen.

Aus der DE-90'02'962.3 U ist eine den Oberbegriff des Patentanspruches 1 bildende Innensohle bekannt. Hierbei sind die Grundschicht und die Erhebungen integral einheitlich aus einem Schaumstoffmaterial mit einer Härte von 30° bis 45° Shore A gefertigt. Vorzugsweise wird diese Innensohle aus Kunststoff gefertigt. Sie kann sowohl als Einlegesohle als auch als Innensohle angeordnet sein. Bei dieser bekannten Innensohle sind sowohl die Grundschicht als auch die Erhebungen aus einem vorzugsweise synthetischen Material gefertigt. Latex als ein Naturprodukt hat klassischerweise eine Härte von Shore A von 10°-20°. Es lässt sich zwar auf grössere Härten einstellen, doch verteuert dies einerseits das Produkt und andererseits sind die Beimischungen teilweise physiologisch unerwünscht.

Aus EP1245167 ist ebenfalls eine den Oberbegriff des Patentanspruches 1 bildende Innensohle bekannt. Hierbei umfasst die Innensohle sowohl eine Grundschicht als auch eine Deckschicht, wobei die Letztere auf der Grundschicht angebracht ist. Die Grundschicht besteht aus einer geschäumten Materialschicht, die eine Härte von vorzugsweise zwischen 10° und 20° Shore A aufweist. Zusammen mit der Deckschicht, weist die Innensohle eine Härte zwischen 30° und 40° Shore A auf. Die einzelnen Erhebungen der Innensohle erbringen somit waehrend des Gehens eine sanfte und wirksame Fussreflexzonenmassage, wobei harte Stösse abgefedert und demzufolge mit verminderter Stärke über die Reflexzonenmassagepunkte auf die Fusssohle übertragen werden. Die Erhebungen der Innensohle im Bereich der Reflexzonen sollte so angepasst sind, dass eine optimale Massagewirkung erwirkt wird. Im Speziellen wird eine effektive Massagewirkung dann erreicht, wenn die Kante der Erhebung auf die Fussreflexzone einwirkt. Da jedoch die Anatomie eines jeden Fusses unterschiedlich ist, und insbesondere die Position beispielsweise des Zehenkopfes der grossen Zehen im Verhältnis zur Innensohle von Person zu Person stark variieren kann, ist eine effektive Massagewirkung nicht für jede Person gewährleistet.

Eine Neupositionierung der Kante beispielsweise mittels einer blossen lateralen Vergrösserung des Durchmessers einer Erhebung, um bei einer dementsprechenden Zehenausrichtung eine Massage zu erwirken, ist nicht immer möglich, da der Innenraum des Schuhes dadurch eingeengt wird und für den Träger deshalb entsprechend unbequem ist. Des Weiteren würde es sich als schwierig und kostspielig erweisen auch nur für eine Auswahl der verschiedensten Fussanatomien Innensohlen mit entsprechend geformten Erhebungen herzustellen.

Die vorliegende Erfindung stellt sich daher die Aufgabe eine Innensohle zu schaffen, welche auch bei verschiedenen Fussanatomien jeweils eine gesicherte effektive Massagewirkung gewährleistet.

Diese Aufgabe löst eine Innensohle mit den Merkmalen des Patentanspruches 1. Dank der mindestens einen zentrischen und umlaufenden Erhöhung der mindestens einen Erhebung im Bereich der Reflexzonen der Fusssohle, weisen dieser Bereich eine Mehrzahl von Kanten auf, von denen mindestens eine beim Gehen akkupressurartig auf eine Fussreflexzone einwirkt.

Weitere vorteilhafte Ausgestaltungsformen gehen aus den abhängigen Ansprüchen hervor und deren Wirkungsweise und Bedeutung ist in der nachfolgenden Beschreibung erläutert. In den Figuren sind bevorzugte Ausführungsbeispiele des Erfindungsgegenstandes dargestellt und nachfolgend im Detail erklärt.

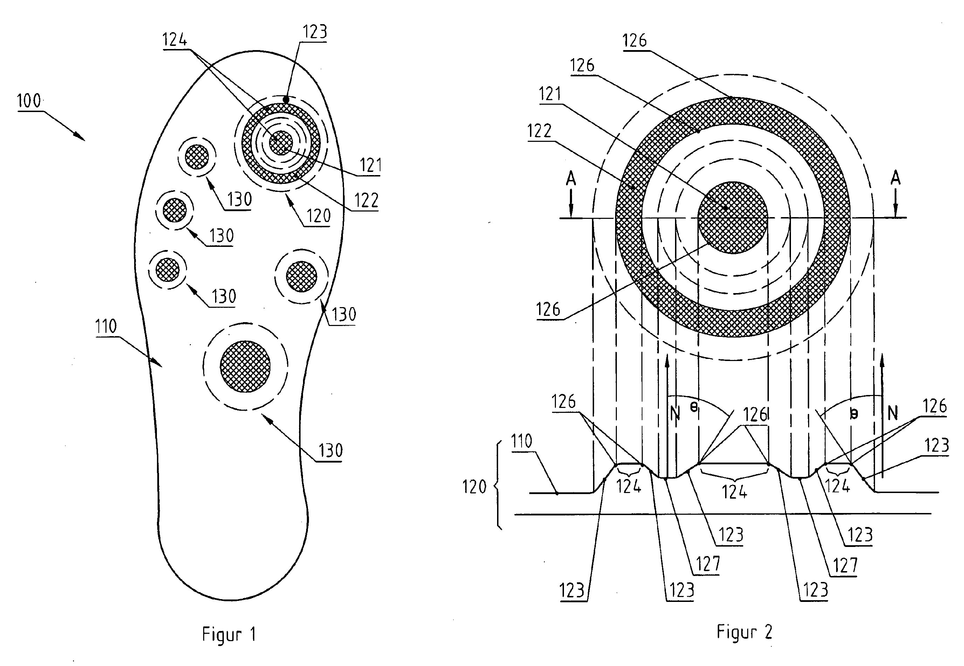

- **Figur 1**: zeigt schematisch eine Aufsicht auf ein erfindungsgemässes Halbfabrikat mit Erhebungen im Bereich von Reflexzonen der Fusssohle in einer Anordung, die zur Einflussnahme auf den Kopfbereich und Sinnesorgane geeignet ist;
- **Figur 2**: zeigt schematisch eine vergrösserte Aufsicht einer erfindungsgemässen Erhebung und den entsprechenden Vertikalschnitt der Erhebung entlang der Linie A-A;
- **Figuren 3-6**: zeigen Ausschnitte von Halbfabrikaten, welche Erhebungen unterschiedlicher Formen zur gezielten Beeinflussung verschiedener Reflexzonen zur Einwirkung auf entsprechende Organe oder Körperteile aufweisen;
- **Figuren 7-8**: zeigen zwei schematisch vergrösserte Querschnitte durch das mit einer Deckschicht versehenen Halbfabrikat, als Einlegesohle oder Innensohle in entsprechenden Ausführungsformen der Erfindung;
- **Figuren 9-15**: zeigen schematisch Aufsichten von Halbfabrikaten beziehungsweise Einlegesohlen oder Innensohlen mit unterschiedlichen Erhebungsformationen zur gezielten Beeinflussung verschiedener Reflexzonen zur Einwirkung auf entsprechende Organe und/oder Körperbereiche; und
- **Figure 16**: zeigt schematisch eine Aufsicht einer besonders bevorzugten Ausführungsform eines Halbfabrikates beziehungsweise einer Einlegesohle mit entsprechenden Erhebungsformationen zur gezielten Beeinflussung verschiedener Reflexzonen zur Einwirkung auf entsprechende Organe und/oder Körperbereiche.
- **Figur 17**: zeigt eine Seitenansicht der Einlegesohle beziehungsweise Innensohle gemäss der Figur 16.

Mit der Bezugszahl **100** ist das Halbfabrikat als Ganzes bezeichnet, welches eine grundsaetzlich ebene Grundfläche **110** aufweist, worauf Erhebungen **120** im Bereich von Reflexzonen angeordnet sind. Die Erhebungen **120** koennen integral mit der Grundfläche **110** gefertigt sein. Das Halbfabrikat **100** kann demzufolge einstückig aus dem gleichen elastischen Schaumstoffmaterial beschaffen sein, beispielsweise aus Latex oder Kunststoff.

In einer Ausführungsform der Erfindung weisen beispielsweise die Erhebung **120** im Bereich des Zehenkopfes mindestens eine zentrierte Erhöhung **121** und eine umlaufende Erhöhung **122** auf. Um die nachfolgende Diskussion zu vereinfachen, sind Ausführungsformen der Erfindung lediglich in Zusammenhang mit der Erhebung **120** im Bereich des Zehenkopfes beschrieben, da wie eingangs erwaehnt, bei verschiedenen Personen die Position eines Zehenkopfes relativ zur Innensohle ziemlich stark variieren kann. Dies sollte jedoch nicht als einschränkend sondern lediglich als beispielhaft interpretiert werden. Ausführungsformen der Erfindung können demzufolge zusätzlich oder alternativ auch in anderen Bereichen von Reflexzonen zur Anwendung kommen, wie zum Beispiel im Bereich des Solarplexus. Einfache Erhebungen, die keine zentrierte und umlaufende Erhöhung aufweisen, wie zum Beispiel in EP1245167 beschrieben, sind mit dem Bezugszeichen **130** bezeichnet.

Die mindestens eine zentrische Erhöhung **121** und umlaufende Erhöhung **122** sind so gestaltet, dass diese jeweils eine von der Grundsohle **110** ausgehende rampenartige seitliche Neigung **123** aufweisen, die in eine ebene Fläche **124** der Erhöhung **121** und **122** mündet, wobei die Neigungen **123** jeweils die ebenen Flächen **124** umlaufen. Diese Gestaltungsweise kann erwirken, dass die Innensohle nicht nur während des Gehens zur Massage der Reflexzonen dient, sonder auch im Ruhezustand eine Akupressurwirkung erzielt, welche relativ sanft wirkt. Die seitlichen Neigungen **123** haben in einer Ausführungsform der Erfindung, gemessen von einer Normalen **N** der Grundflaeche **110,** einen Winkel θ zwischen 30° und 60°.

Die mindestens eine zentrierte Erhöhung **121** und umlaufende Erhöhung **122** formen eine Mehrzahl von erhöhten Massagekanten **126** und Vertiefungen **127** im Bereich der Erhebung **120** der Reflexzone. Diese Mehrzahl von Massagekanten **126** erwirken bei Zehenköpfen in verschiedenen Positionen auf der Erhebung **120** eine Akkupressur. Im Normalfall sind sämtliche Erhebungen **120** und **130** gleich stark über der Grundfläche **110** nach oben vorstehend.

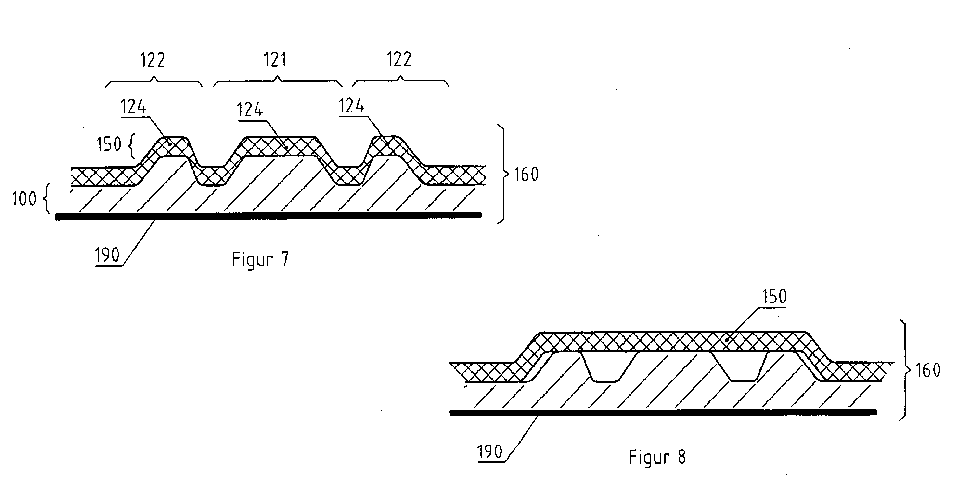

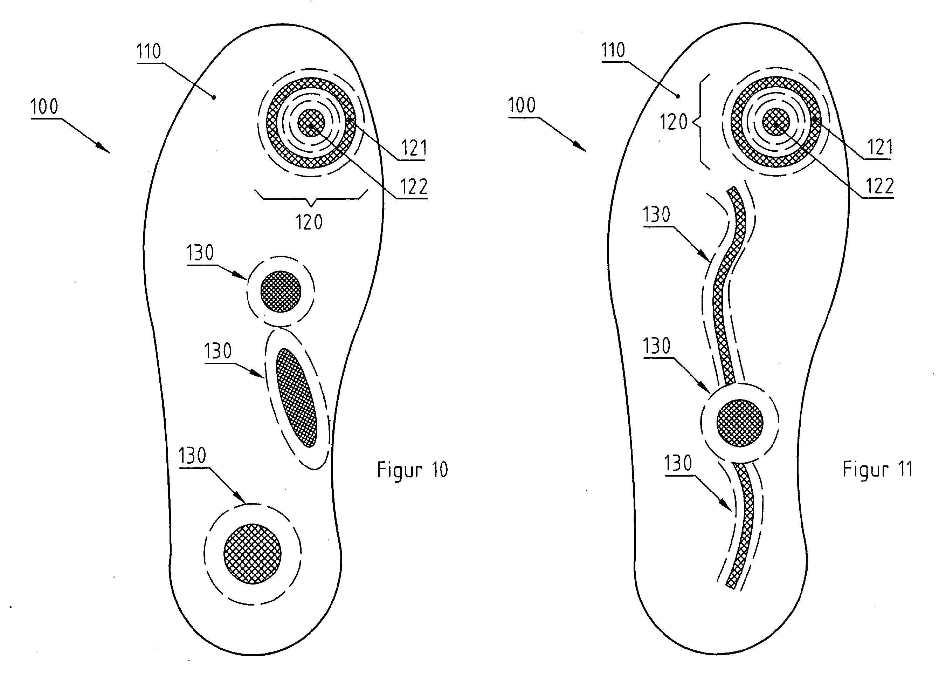

Die zentrierten Erhöhungen **121** brauchen keineswegs lediglich eine kreisrunde Gestalt (z. Bsp. einer Noppe) zu haben sondern können auch, wie in den **Figuren 7-10** schematisch dargestellt, beliebige andere Ausgestaltungsformen haben und zum Beispiel, ovalartig oder linienförmig verlaufen. Eine linienförmige Erhebung **130** beispielsweise kann eine Scheitellinie darstellen, die auf mindestens annähernd gleichbleibender Höhe verläuft. Insbesondere kann die Erhebung **120,** wie in **Figuren 7-9** schematisch dargestellt, mehrere zentrierte Erhöhungen **121** aufweisen, welche wiederum unterschiedliche Grundrisse haben können.

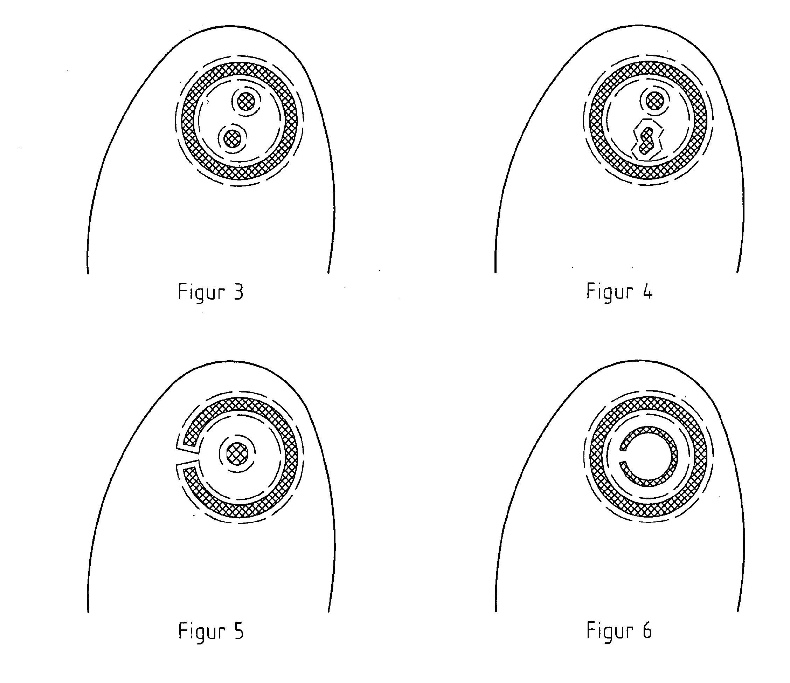

Wie in **Figur 5** schematisch dargestellt, kann die mindestens eine umlaufende Erhöhung **122** sowohl geschlossen als auch offen die mindestens eine zentrierte Erhöhung **121** umlaufen.

Die zuvor beschriebenen Halbfabrikate dienen zur Weiterverarbeitung zu Innensohlen oder Einlegesohlen. Diese bestehen aus mindestens drei Lagen, nämlich der weichen aus geschäumtem Material, insbesondere Latex oder Kunststoff gefertigte Massagelage **100,** die zuvor als Halbfabrikat **100** beschrieben worden ist, sowie einer oberen Decksicht **150** und einer unteren Tragschicht **190.** Diese drei Schichten werden durch entsprechende Kaschierung oder anderer Art der Verklebung miteinander verbunden.

Die Wahl der Materialien ist herkömmlich. So wird die Decksicht vorzugsweise aus einem atmungsaktiven Material wie Leder oder Textil gefertigt sein. Für preiswertere Lösungen wird man vorzugsweise auch Kunstleder als Deckschicht einsetzen.

Die Tragschicht kann mehrere Funktionen erfüllen. Die Massageschicht ist kaum abreibfest und muss daher geschützt werden. Diese Funktion erfüllt die Deckschicht. Gleichzeitig kann Sie weitere Funktionen erfüllen, wie stossdämpfend wirken, dampfdurchlässig sein, thermisch isolierend wirken. Diese Funktionen erfüllen diverse auf dem Markt für die Schuhindustrie angebotenen Materialen aus Kunststoff oder auf Zellulosebasis gefertigte Folien /z.B. unter der Marke Texon).

Wie in **Figur 7** schematisch dargestellt, kann die Erhebung **120** beispielsweise vollständig mit der Deckschicht **150** versehen sein und auch vollflächig verklebt, wobei die Deckschicht **150** den Konturen der Massageschicht beziehungsweise des Halbfabrikates vollständig folgt.

In **Figur 8** ist eine Ausführungsform der Erfindung dargestellt, in der die Deckschicht **150** durchgehend eben über die Erhebung **120** angeordnet ist, so dass zwischen der Deckschicht **150** und der mindestens einen Vertiefung **127** mindestens ein Hohlraum geformt ist. Die Deckschicht **150** folgt somit nur den äussersten Konturen der Erhebungen und nicht den Konturen der Zwischenvertiefungen. Dies reduziert Scherbewegungen auf die Massagelage beziehungsweise auf das Halbfabrikat, wodurch die Lebensdauer des Fertigproduktes, nämlich der Innensohle oder der Einlegesohle, erhöht wird, ohne dass die Massagewirkung reduziert wird.

In einer bevorzugten Ausführungsform der Erfindung weist das elastische Schaumstoffmaterial des Halbfabrikats **100** eine Härte von unter 25° Shore A und insbesondere zwischen 10° und 20° Shore A aufweist, so dass die Härte der mit der Deckschicht **150** versehenen Innensohle oder Einlegesohle **160** eine Härte von über 25° Shore A, und insbesondere zwischen 30° und 40° Shore A aufweist.

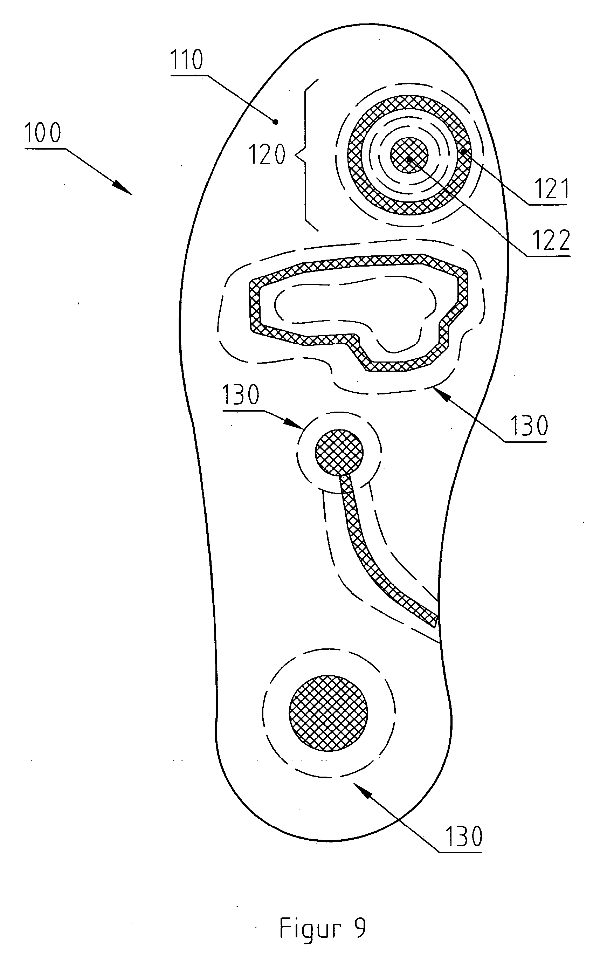

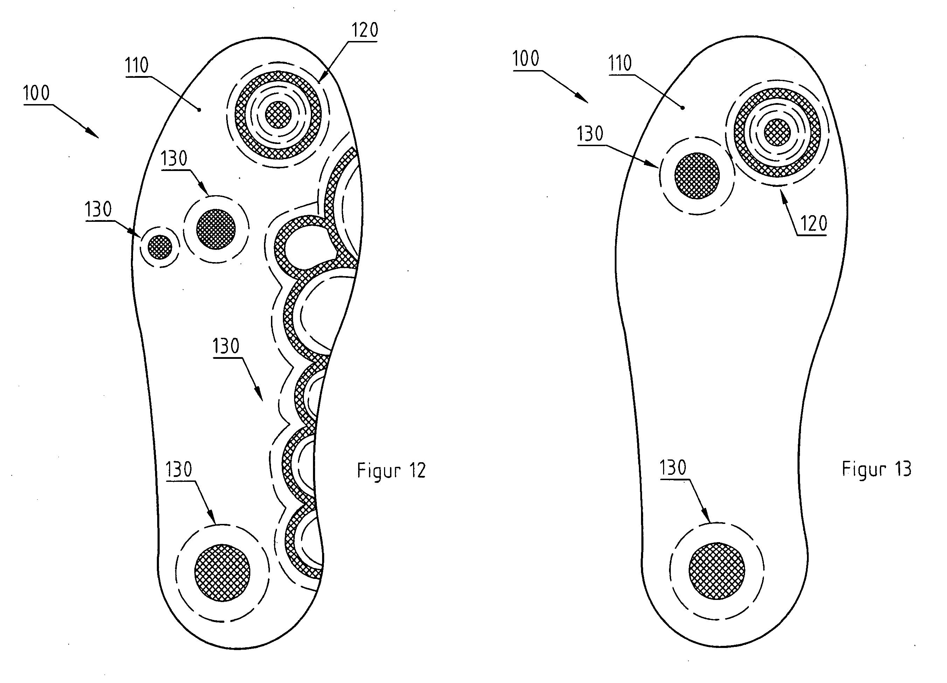

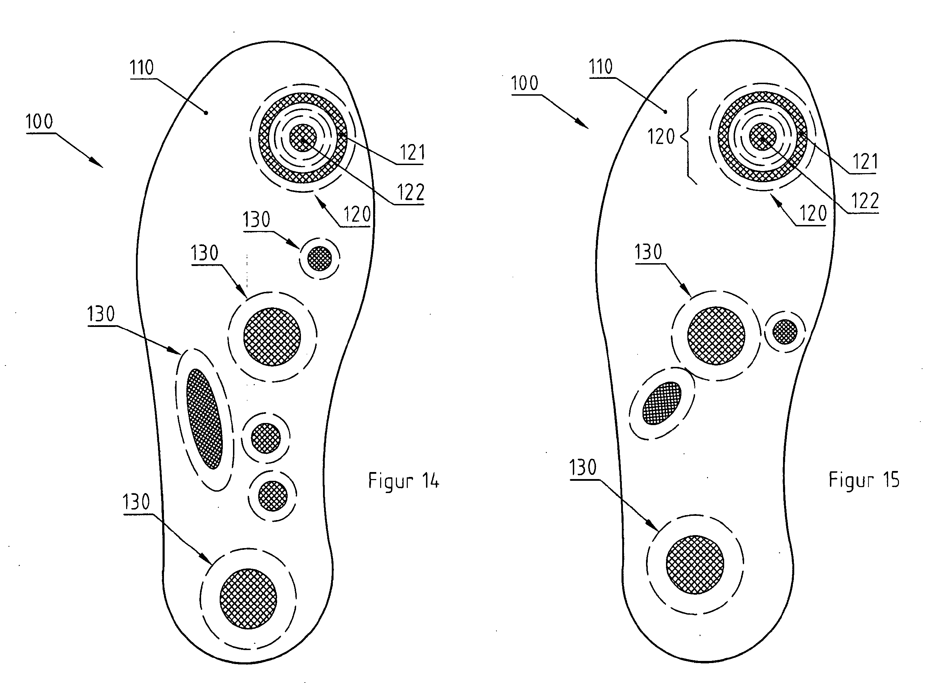

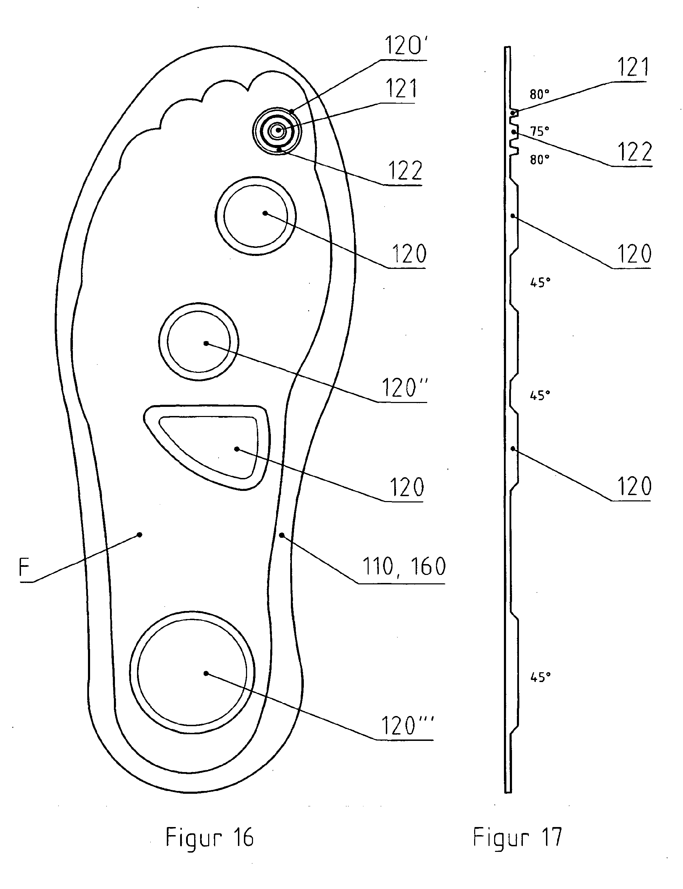

Die Figuren 9 - 16 zeigen Innensohlen oder Einlegesohlen mit unterschiedlichen Formen der Erhebungen 120. Je nach der Anordnung und Form lassen sich verschiedene Organe und Körperpartien in Kombination stimulieren. Besonders bevorzugt ist eine an sich bekannte Ausführungsform, die als "5-Punkt" oder "5-Zonen" Sohlen bekannt ist, und in der Figur 16 dargestellt ist. Eine solche Sohle dient der Verbesserung des allgemeinen Wohlgefühls und ist für die meisten Personen anwendbar. In der Figur 16 ist einerseits der Umriss der Innensohle oder Einlegesohle **160** dargestellt und darauf der Umriss eines Fusses abgebildet. Während der Fersenpunkt durch die Schuhform festgelegt ist und somit nicht variiert, können die anderen Punkte bezüglich ihrer Lage etwas variieren.

Am stärksten veränderbar in der Lage ist der Zehenpunkt. Je nach Person steht der grosse Zeh mehr oder weniger ab oder ist nach innen geneigt verlaufend. Da für die Stimulation die Kanten der Erhebungen **120** wirksam sind, wird durch die erfindungsgemässe Ausgestaltung der Erhebung **120'** insbesondere für den grossen Zehen mit einer zentrischen Erhöhnung **121** und einer umlaufenden Erhöhung 122 und eine Vielzahl über die gesamte Fläche der Erhebung **120'** verteilte wirksame obere Massagekanten **126** gebildet, von denen praktisch unabhängig der Verlaufsrichtung des grossen Zehens mindestens ein Teil der oberen Massagekanten **126** wirksam sind.

Neben der Massagezone für den grossen Zehen können auch die Erhebungen für die anderen Massagezonen gleich ausgestaltet sein. Insbesondere kommt hier die Erhebung **120"** zur Stimulierung des Sonnengeflechtes in Frage. Diese Erhebung ist in der Figur 16 mit **120'** gekennzeichnet, jedoch in der Figur der Einfachheithalber als herkömmlich gestaltete Erhebung gezeichnet.

In der Figur 17 erkennt man die Innensohle beziehungsweise Einlegesohle nach Figur 16 in der Seitenansicht. Um die Stimulation zu erhöhen sind die seitlichen Neigungen, insbesondere bei den erfindungsgemäss gestalteten Erhebungen **120,** mit steileren Neigungen relativ zur Grundfläche **110** versehen als bei der herkömmlich gestalteten Erhebungen. Bevorzugterweise sind die Neigungen der Seitenkanten der Erhebungen bei herkömmlichen Erhebungen **120** mit einem Neigungswinkel von rund 45° versehen. Die Neigung der umlaufenden Erhöhung **122** hat einen Neigungswinkel von 80°, während der Neigungswinkel der zentrischen Erhöhung 121 75° beträgt. Diese Neigungen sind bevorzugte Angaben und betreffen die Neigung wie sie bei dem unkaschierten Halbfabrikat **100** realisiert werden. Diese Neigungswinkel sind nach der Kaschierung mit einer oberen Deckschicht **150** automatisch etwas verändert und meist etwas flacher. Nach einer gewissen Tragzeit wird sich dies nochmals ändern. Insbesondere bei steilen Neigungswinkeln kann es zu Faltenbildung kommen. Dies ist jedoch keineswegs nachteilig, sondern führt zu einer nochmals erhöhten Stimulation.

### Bezugszeichenliste

- 100: Halbfabrikat
- 110: Grundfläche
- 120: Erhebungen
- 121: zentrische Erhöhung
- 122: umlaufende Erhöhung
- 123: seitliche Neigung
- 124: ebene Fläche
- 126: erhöhte Massagekanten
- 127: Vertiefungen
- 130: linienförmige Erhebung
- 150: obere Deckschicht
- 160: Innensohle oder Einlegesohle
- 190: untere Tragschicht
- F: Fussumriss

## Patentansprüche

1. Halbfabrikat (100) zur Fertigung einer Innensohle (160), bestehend aus einer dem Umriss eines Schuhs entsprechenden, elastischem Schaumstoffmateriallage, die auf ihrer oberen Seite, von der Grundfläche (110) des Halbfabrikats (100) ausgehend, mehrere ebenfalls aus demselben elastischen Schaumstoffmaterial bestehende, integral angeformte Erhebungen (120) im Bereich von Reflexzonen der Fusssohle aufweist, **dadurch gekennzeichnet, dass** mindestens die den Zehenkopf des grossen Zehens massierende Erhebung (120) mindestens eine zentrische und mindestens eine umlaufende Erhöhung (122) aufweisen, welche eine Mehrzahl von Massagekanten formen.

2. Halbfabrikat (100) nach Anspruch 1, **dadurch gekennzeichnet, dass** sowohl die mindestens eine zentrische Erhöhung (121) und umlaufende Erhöhung (122) von der Grundsohle ausgehende rampenartige seitliche Neigungen (123) aufweisen, die in eine flächige Erhebung (120) münden.

3. Halbfabrikat (100) nach einem der vorherigen Ansprüche, **dadurch gekennzeichnet, dass** gemessen von einer Normalen der Grundfläche (110), die seitliche Neigung (123) einen Winkel zwischen 30° und 60° aufweist.

4. Halbfabrikat (100) nach einem der vorherigen Ansprüche, **dadurch gekennzeichnet, dass** die mindestens eine zentrische Erhöhung (121) und mindestens eine umlaufende Erhöhung (122), die nebeneinander angeordnet sind, mindestens eine entsprechende Vertiefung (127) formen.

5. Halbfabrikat (100) nach Anspruch 1, **dadurch gekennzeichnet, dass** nur der den Zehenkopf des grossen Zehens massierende Erhebung (120) mit mindestens einer umlaufenden Erhöhung (122) versehen ist.

6. Halbfabrikat (100) nach Anspruch 5, **dadurch gekennzeichnet, dass** zusätzlich die der Massage des Solar Plexus dienende Erhebung (120) mit mindestens einer umlaufenden Erhöhung (122) versehen ist.

7. Innensohle oder Einlegesohle (160) gefertigt aus einem Halbfabrikat (100) nach einem der vorherigen Ansprüche, **dadurch gekennzeichnet, dass** die geschäumte Materialschicht des Halbfabrikates (100) als Massagelage zwischen einer Deckschicht (150) und einer Tragschicht (190) angeordnet ist.

8. Innensohle oder Einlegesohle (160) nach Anspruch 7, **dadurch gekennzeichnet, dass** die Deckschicht (150) aus Leder oder Textilmaterial gefertigt ist.

9. Innensohle oder Einlegesohle (160) nach Anspruch 7, **dadurch gekennzeichnet, dass** die Tragschicht (190) aus einem Material auf Zellulosebasis oder aus Kunststoff gefertigt ist.

10. Innensohle oder Einlegesohle (160) nach Anspruch 7, **dadurch gekennzeichnet, dass** die Deckschicht (150), der Konturen des Halbfabrikates (100) als Massagelage vollflächig folgend, verbunden ist.

11. Innensohle oder Einlegesohle (160) nach Anspruch 7, **dadurch gekennzeichnet, dass** die Deckschicht (150), die nur den äusseren Konturen der Erhebungen folgt, und die Zwischenräume zwischen der zentrischen Erhöhung und der mindestens einen umlaufenden Erhöhung überbrückt unter Freilassung eines Hohlraumes.

12. Innensohle oder Einlegesohle (160) nach Anspruch 7, **dadurch gekennzeichnet, dass** das elastische Schaumstoffmaterial der Massagelage eine Härte von unter 25° Shore A aufweist, so dass die Härte der mit der Deckschicht (150) versehenen Innensohle (160) eine Härte von über 25° Shore A aufweist.

13. Innensohle oder Einlegesohle nach Anspruch 12, **dadurch gekennzeichnet, dass** das elastische Schaumstoffmaterial der Massagelage eine Härte von zwischen 10° und 20° Shore A aufweist.

14. Innensohle oder Einlegesohle nach Anspruch 12, **dadurch gekennzeichnet, dass** die Härte der mit der Deckschicht versehenen Innensohle oder Einlegesohle (160) eine Härte von zwischen 30° und 40° Shore A aufweist.

## Claims

1. A semi-finished product (100) for fabricating an insole (160) consisting of an elastic foam material layer corresponding to the outline of a shoe, which on its upper side, starting from the base surface (110) of the semi-finished product (100), has a plurality of integrally formed raised sections (120), likewise consisting of the same elastic foam material, in the region of reflex zones of the sole of the foot, **characterized in that** at least the raised section (120) which massages the toe head of the big toe comprises at least one central and at least one circumferential elevation (122) which form a plurality of massage edges.

2. The semi-finished product (100) according to claim 1, **characterized in that** both the at least one central elevation (121) and circumferential elevation (122) have ramp-like lateral inclinations (123) starting from the basic sole, which open into a flat raised section (120).

3. The semi-finished product (100) according to any one of the preceding claims, **characterized in that** measured from a normal to the base surface (110), the lateral inclination (123) has an angle between 30° and 60°.

4. The semi-finished product (100) according to any one of the preceding claims, **characterized in that** the at least one central elevation (121) and at least one circumferential elevation (122) which are disposed adjacently to one another, form at least one corresponding recess (127).

5. The semi-finished product (100) according to claim 1, **characterized in that** only the raised section (120) which massages the toe head of the big toe is provided with at least one circumferential elevation (122).

6. The semi-finished product (100) according to claim 5, **characterized in that** in addition, the raised section used to massage the solar plexus (120) is provided with at least one circumferential elevation (122).

7. An insole or shoe insert (160) fabricated from a semi-finished product (100) according to any one of the preceding claims, **characterized in that** the foamed material layer of the semi-finished product (100) is disposed as a massage layer between a cover layer (150) and a supporting layer (190).

8. The insole or shoe insert (160) according to claim 7, **characterized in that** the cover layer (150) is made from leather or textile material.

9. The insole or shoe insert (160) according to claim 7, **characterized in that** the supporting layer (190) is fabricated from a cellulose-based material or from plastic.

10. The insole or shoe insert (160) according to claim 7, **characterized in that** the cover layer (150) is connected as a massage layer extensively following the contours of the semi-finished product (100).

11. The insole or shoe insert (160) according to claim 7, **characterized in that** the cover layer (150) only follows the outer contours of the raised sections and bridges the intermediate spaces between the central elevation and the at least one circumferential elevation leaving free a cavity.

12. The insole or shoe insert (160) according to claim 7, **characterized in that** the elastic foam material of the massage layer has a hardness of less than 25° Shore A so that the hardness of the insole (160) provided with the cover layer (150) has a hardness of higher than 25° Shore A.

13. The insole or shoe insert according to claim 12, **characterized in that** the elastic foam material of the massage layer has a hardness of between 10° and 20° Shore A.

14. The insole or shoe insert according to claim 12, **characterized in that** the hardness of the insole or shoe insert (160) provided with the cover layer has a hardness of between 30° and 40° Shore A.

## Revendications

1. Produit semi-fini (100) pour la fabrication d'une semelle intérieure (160), consistant en une couche de mousse élastique correspondant au contour d'une chaussure, qui présente, sur sa face supérieure, en partant de la surface de base (110) du produit semi-fini (100), plusieurs proéminences (120) formées en faisant bloc et également composées de la même mousse élastique au niveau des zones réflexes de la plante du pied, **caractérisé en ce qu'au** moins la proéminence (120) massant la tête du gros orteil présente au moins une proéminence centrée et au moins une proéminence périphérique (122) qui forment une pluralité de bords massants.

2. Produit semi-fini (100) selon la revendication 1, **caractérisé en ce qu'aussi** bien l'au moins une proéminence centrée (121) que la proéminence périphérique (122) présentent des pentes latérales en forme de rampes (123) partant de la semelle de base et qui débouchent dans une proéminente plane (120).

3. Produit semi-fini (100) selon une des revendications précédentes, **caractérisé en ce que,** mesurée depuis une normale de la surface de base (110), la pente latérale (123) décrit un angle de 30° à 60°.

4. Produit semi-fini (100) selon une des revendications précédentes, **caractérisé en ce que** l'au moins une proéminence centrée (120) et l'au moins une proéminence périphérique (122) qui sont juxtaposées forment un creux correspondant (127).

5. Produit semi-fini (100) selon la revendication 1, **caractérisé en ce que** seule la proéminence (120) massant la tête du gros orteil est pourvue d'au moins une proéminence périphérique (122).

6. Produit semi-fini (100) selon la revendication 5, **caractérisé en ce que** la proéminence (120) servant à masser le plexus solaire est en plus pourvue d'au moins une proéminence périphérique (122).

7. Semelle intérieure ou semelle à insérer (160) fabriquée à partir d'un produit semi-fini (100) selon une des revendications précédentes, **caractérisée en ce que** la couche de mousse du produit semi-fini (100) est disposée sous forme de couche massante entre une couche de recouvrement (150) et une couche porteuse (190).

8. Semelle intérieure ou semelle à insérer (160) selon la revendication 7, **caractérisée en ce que** la couche de recouvrement (150) est réalisée en cuir ou en textile.

9. Semelle intérieure ou semelle à insérer (160) selon la revendication 7, **caractérisée en ce que** la couche porteuse (190) est faite d'un matériau à base de cellulose ou de plastique.

10. Semelle intérieure ou semelle à insérer (160) selon la revendication 7, **caractérisée en ce que** la couche de recouvrement (150) est raccordée sous forme d'une couche massante suivant les contours du produit semi-fini (100) sur toute sa surface.

11. Semelle intérieure ou semelle à insérer (160) selon la revendication 7, **caractérisée en ce que** la couche de recouvrement (150) qui suit seulement les contours extérieurs des proéminences chevauche les intervalles entre la proéminence centrée et l'au moins une proéminence périphérique en dégageant une cavité.

12. Semelle intérieure ou semelle à insérer (160) selon la revendication 7, **caractérisée en ce que** la mousse élastique de la couche massante présente une dureté de moins de 25° Shore A, de sorte que la dureté de la semelle intérieure (160) pourvue de la couche de recouvrement (150) présente une dureté de plus de 25° Shore A.

13. Semelle intérieure ou semelle à insérer selon la revendication 12, **caractérisée en ce que** la mousse élastique de la couche massante présente une dureté de 10° à 20° Shore A.

14. Semelle intérieure ou semelle à insérer selon la revendication 12, **caractérisée en ce que** la dureté de la semelle intérieure (160) pourvue de la couche de recouvrement présente une dureté de 30° à 40° Shore A.
